(19) Europäisches Patentamt
European Patent Office
Office européen des brevets

(11) Publication number: **0 366 273 A1**

(12) # EUROPEAN PATENT APPLICATION

(21) Application number: 89309927.5

(22) Date of filing: 28.09.89

(51) Int. Cl.5: C07D 311/68 , C07D 217/04 , C07D 217/08 , C07D 405/04 , C07D 407/04 , C07D 491/052 , C07D 491/04 , A61K 31/44 , //(C07D405/04,311:00,207:00)

The title of the invention has been amended (Guidelines for Examination in the EPO, A-III, 7.3).

(30) Priority: 28.09.88 GB 8822743

(43) Date of publication of application: 02.05.90 Bulletin 90/18

(84) Designated Contracting States: BE CH DE FR GB IT LI NL

(71) Applicant: BEECHAM GROUP PLC
Beecham House Great West Road
Brentford Middlesex TW8 9BD(GB)

(72) Inventor: Frydrych, Catherine S.V. Beecham Pharmaceuticals
Great Burgh Yew Tree Bottom Road
Epsom Surrey KT18 5XQ(GB)
Inventor: Evans, John Morris Beecham Pharmaceuticals
Coldharbour Road The Pinnacles
Harlow Essex CM19 5AD(GB)

(74) Representative: Rutter, Keith et al
Beecham Pharmaceuticals Great Burgh Yew Tree Bottom Road
Epsom Surrey KT18 5XQ(GB)

(54) **Benzopyran derivatives with smooth muscle relaxant activity.**

(57) A compound of formula (I):

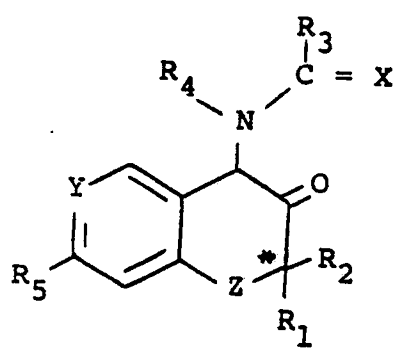

(I)

or, where appropriate, a pharmaceutically acceptable salt thereof, or a pharmaceutically acceptable solvate thereof
wherein:
X represents oxygen or sulphur;
Y represents N or $N^{+}\text{-}O^{-}$ or a moiety $CR_6$ wherein $R_6$ is as defined below;
Z represents O, $-CH_2-$, $NR^o$, a bond or $S(O)_p$ wherein p represents zero, 1 or 2;

EP 0 366 273 A1

$R^o$ represents hydrogen, alkyl or alkylcarbonyl;
$R_1$ and $R_2$ independently represent hydrogen or alkyl; or
$R_1$ and $R_2$ together represent a $C_{2-7}$ polymethylene moiety;
$R_3$ represents hydrogen; alkyl optionally substituted by one or more groups or atoms selected from halogen, hydroxy or an ester thereof, alkoxy, alkoxycarbonyl, carboxy or an ester or amide thereof, amino, monoalkylamino or dialkylamino; alkenyl; amino optionally substituted by an alkyl or alkenyl group or by an alkanoyl group optionally substituted by up to three halo atoms, by a phenyl group optionally substituted by alkyl, alkoxy or halogen; substituted or unsubstituted aryl or substituted or unsubstituted heteroaryl; and
$R_4$ represents hydrogen or alkyl;
or $R_3$ and $R_4$ together represent a linking chain of formula $-A^1-A^2-$, $A^1$ being attached to the nitrogen atom of the moiety -N-CX- and $A^2$ being attached to the carbon atom of the group CX on the said moiety, and wherein $A^1$ represents a substituted or unsubstituted methylene group, $A^2$ represents 2 or 3 linking members, one of the linking members optionally representing O, S or NR and the other linking members each independently representing a substituted or unsubstituted methylene group;
R represents hydrogen, alkyl, alkanoyl, phenyl $C_{1-4}$-alkyl, arylcarbonyl wherein the aryl group may be substituted or unsubstituted; or R is mono- or bi-cyclic- heteroarylcarbonyl; or the moiety $R_4$.N.CX.$R_3$ forms a substituted or unsubstituted 2-pyridonyl group or a substituted or unsubstituted 2-thiopyridonyl group;
$R_5$ and $R_6$ are each independently selected from the class of hydrogen, substituted or unsubstituted alkyl, alkoxy, fluoroalkoxy, $C_{3-8}$ cycloalkyl, hydroxy or an ester thereof, nitro, cyano, halo, formyl, carboxy, a group of formula $R^aT^1$-, $R^bR^cNT$-, $R^aT^2NH$-, $R^dCO.O$-, $R^dCS.O$-, $R^d(OH)CH$-, $R^d(SH)CH$-, $R^dC(=N.OH)$-, $R^dC(=N.NH_2)$- or alkenyl optionally substituted by alkylcarbonyl, nitro or cyano, providing that when Y is $CR_6$ then at least one of $R_5$ or $R_6$ is not hydrogen;
$R^a$ represents $R^d$ or $R^dO$- and $R^d$ represents substituted or unsubstituted alkyl, substituted or unsubstituted aryl or substituted or unsubstituted heteroaryl, optional substituents for $R^d$ being up to 3 substituents selected from alkyl, alkoxy, halo, haloalkyl, nitro and cyano;
$R^b$ and $R^c$ each independently represent hydrogen, alkyl or alkylcarbonyl;
T represents a bond or $T^1$, $T^1$ represents -CS- or $T^2$ and
$T^2$ represents -CO-, -SO- or $-SO_2$-; a process for preparing such a compound, a pharmaceutical composition comprising such a compound and the use of such a compound or composition in medicine.

# NOVEL COMPOUNDS

This invention relates to certain heterocyclyl derivatives to processes for their preparation, to compositions containing such compounds and to the use of such compounds and compositions in medicine.

EP-A-76075, 93535, 95316, 107423, 120426, 126311, 126350, 126367 and 138134 describe certain benzopyran derivatives having inter alia antihypertensive activity. EP-A-176689 also discloses that certain benzopyran derivatives are useful for the treatment of inter alia disorders of the respiratory system.

A group of novel heterocyclyl derivatives has now been discovered which surprisingly has smooth muscle relaxant activity, and such compounds are therefore potentially useful as bronchodilators in the treatment of disorders of the respiratory tract, such as reversible airways obstruction and asthma, and also in the treatment of hypertension. Such compounds are also indicated to be of potential use in the treatment of disorders associated with smooth muscle contraction of the gastro-intestinal tract, uterus or the urinary tract including the ureter. Such disorders respectively include irritable bowel syndrome and diverticular disease; premature labour; incontinence; renal cholic and disorders associated with the passage of kidney stones. They are also indicated as of potential use in the treatment of cardiovascular disorders other than hypertension, such as congestive heart failure, angina, peripheral vascular disease and cerebral vascular disease; and also in the treatment and/or prophylaxis of disorders associated with pulmonary hypertension and of disorders associated with right heart failure.

Accordingly, the present invention provides a compound of formula (I):

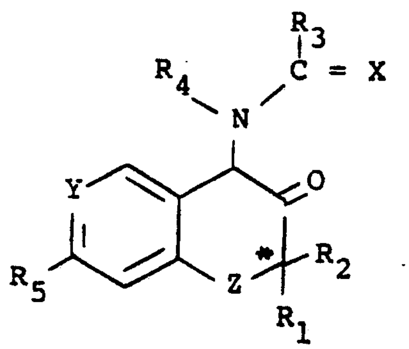

(I)

or, where appropriate, a pharmaceutically acceptable salt thereof, or a pharmaceutically acceptable solvate thereof wherein:

X represents oxygen or sulphur;

Y represents N or $N^{+}$-$O^{-}$ or a moiety $CR_6$ wherein $R_6$ is as defined below;

Z represents O, $-CH_2-$, $NR^o$, a bond or $S(O)_p$ wherein p represents zero, 1 or 2;

$R^o$ represents hydrogen, alkyl or alkylcarbonyl;

$R_1$ and $R_2$ independently represent hydrogen or alkyl; or

$R_1$ and $R_2$ together represent a $C_{2-7}$ polymethylene moiety;

$R_3$ represents hydrogen; alkyl optionally substituted by one or more groups or atoms selected from halogen, hydroxy or an ester thereof, alkoxy, alkoxycarbonyl, carboxy or an ester or amide thereof, amino, monoalkylamino or dialkylamino; alkenyl; amino optionally substituted by an alkyl or alkenyl group or by an alkanoyl group optionally substituted by up to three halo atoms, by a phenyl group optionally substituted by alkyl, alkoxy or halogen; substituted or unsubstituted aryl or substituted or unsubstituted heteroaryl; and

$R_4$ represents hydrogen or alkyl;

or $R_3$ and $R_4$ together represent a linking chain of formula $-A^1-A^2-$, $A^1$ being attached to the nitrogen atom of the moiety -N-CX- and $A^2$ being attached to the carbon atom of the group CX on the said moiety, and wherein $A^1$ represents a substituted or unsubstituted methylene group, $A^2$ represents 2 or 3 linking members, one of the linking members optionally representing O, S or NR and the other linking members each independently representing a substituted or unsubstituted methylene group;

R represents hydrogen, alkyl, alkanoyl, phenyl $C_{1-4}$-alkyl, arylcarbonyl wherein the aryl group may be substituted or unsubstituted; or R is mono- or bi-cyclic- heteroarylcarbonyl; or the moiety $R_4$.N.CX.$R_3$ forms a substituted or unsubstituted 2-pyridonyl group or a substituted or unsubstituted 2-thiopyridonyl group;

$R_5$ and $R_6$ are each independently selected from the class of hydrogen, substituted or unsubstituted alkyl, alkoxy, fluoroalkoxy, $C_{3-8}$ cycloalkyl, hydroxy or an ester thereof, nitro, cyano, halo, formyl, carboxy, a

group of formula $R^aT^1$-, $R^bR^cNT$-, $R^aT^2NH$-, $R^dCO.O$-, $R^dCS.O$-, $R^d(OH)CH$-, $R^d(SH)CH$-, $R^dC(=N.OH)$-, $R^dC(=N.NH_2)$- or alkenyl optionally substituted by alkylcarbonyl, nitro or cyano, providing that when Y is $CR_6$ then at least one of $R_5$ or $R_6$ is not hydrogen;

$R^a$ represents $R^d$ or $R^dO$- and $R^d$ represents substituted or unsubstituted alkyl, substituted or unsubstituted aryl or substituted or unsubstituted heteroaryl, optional substituents for $R^d$ being up to 3 substituents selected from alkyl, alkoxy, halo, haloalkyl, nitro and cyano;

$R^b$ and $R^c$ each independently represent hydrogen, alkyl or alkylcarbonyl;

T represents a bond or $T^1$, $T^1$ represents -CS- or $T^2$ and

$T^2$ represents -CO-, -SO- or $-SO_2-$.

Suitable substituents for any aryl or heteroaryl group represented by $R_3$ include one or more groups or atoms selected from alkyl, alkoxy, hydroxy, halogen, fluoroalkyl, nitro, cyano, carboxy or an ester thereof, alkylcarbonyloxy, amino, monoalkylamino, dialkylamino, aminocarbonyl, monoalkylaminocarbonyl or dialkylaminocarbonyl.

When the linking chain $-A^1-A^2-$ comprises substituted methylene groups it is favoured if one or two of methylene groups are substituted, in particular it is favoured if the methylene group represented by $-A^1-$ is substituted.

Suitable substituents for any methylene group in $-A^1-A^2-$ include alkyl groups, especially methyl or ethyl and in particular methyl.

In one particular aspect, the linking chain $-A^1-A^2-$(and thus $R_3$ and $R_4$ together) represent a moiety of formula $-CH_2-(CH_2)_n-Z'-(CH_2)_m-$ wherein m and n are 0 to 2 such that m + n is 1 or 2 and $Z'$ is $CH_2$, O, S or NR wherein R is as defined above.

Suitably R represents hydrogen, $C_{1-6}$ alkyl, $C_{2-7}$ alkanoyl, phenyl-$C_{1-4}$- alkyl, naphthylcarbonyl, phenylcarbonyl or benzylcarbonyl optionally substituted in the phenyl or naphthyl ring by one or two of $C_{1-6}$ alkyl, $C_{1-6}$ alkoxy or halogen; or R is mono- or bi-cyclic- heteroarylcarbonyl.

In one aspect the moiety $R_4$.N.CX.$R_3$ represents 2-pyridon-1-yl.

A favoured substituent for the pyridonyl or thiopyridonyl group is a $C_{1-6}$ alkyl or aryl group.

Suitably, when Y is N or $N^+-O^-$, $R_5$ is hydrogen.

Suitably, when Y is $CR_6$, then one of $R_5$ and $R_6$ is hydrogen.

In particular, when Y is $CR_6$, one of $R_5$ and $R_6$ is hydrogen and the other is selected from substituted or unsubstituted alkyl, alkoxy, fluoroalkoxy, $C_{3-8}$ cycloalkyl, hydroxy, nitro, cyano, halo, formyl, carboxy, a group of formula $R^aT^1$-, $R^bR^cNT$-, $R^aT^2NH$-, $R^dCO.O$-, $R^dCS.O$-, $R^d(OH)CH$-, $R^d(SH)CH$-, $R^dC(=N.OH)$-, $R^dC(=N.NH_2)$- or alkenyl optionally substituted by alkylcarbonyl, nitro or cyano, wherein $R^a$, $R^b$, $R^c$, $R^d$, T, $T^1$ and $T^2$ are as defined in relation to formula (I).

Also to be mentioned are those compounds wherein one of $R_5$ and $R_6$ is hydrogen and the other is selected from the class of $C_{1-6}$ alkylcarbonyl, $C_{1-6}$ alkoxycarbonyl, $C_{1-6}$ alkylcarbonyloxy, $C_{1-6}$ alkylhydroxymethyl, nitro, cyano, chloro, trifluoromethyl, $C_{1-6}$ alkylsulphinyl, $C_{1-6}$ alkylsulphonyl, $C_{1-6}$ alkoxysulphinyl, $C_{1-6}$ alkoxysulphonyl, $C_{1-6}$ alkylcarbonylamino, $C_{1-6}$ alkoxycarbonylamino, $C_{1-6}$ alkyl-thiocarbonyl, $C_{1-6}$ alkoxy-thiocarbonyl, $C_{1-6}$ alkyl-thiocarbonyloxy, $C_{1-6}$ alkyl-thiomethyl, formyl or aminosulphinyl, aminosulphonyl or aminocarbonyl, the amino moiety being optionally substituted by one or two $C_{1-6}$ alkyl groups, or $C_{1-6}$ alkylsulphinylamino, $C_{1-6}$ alkylsulphonylamino $C_{1-6}$ alkoxysulphinylamino or $C_{1-6}$ alkoxysulphonylamino or ethenyl terminally substituted by $C_{1-6}$ alkylcarbonyl, nitro or cyano, or $-C(C_{1-6}$ alkyl)NOH or $-C(C_{1-6}$ alkyl)$NNH_2$.

In a further particular aspect, $R_5$ represents hydrogen and $R_6$ represents alkyl or $C_{3-8}$ cycloalkyl, especially alkyl.

Suitably, one of $R_5$ and $R_6$ represents nitro, cyano or alkylcarbonyl and the other represents a different group selected from nitro, cyano, halo, alkylcarbonyl, alkoxy, especially methoxy, mono- or di-alkylamino or mono- or di- alkylcarbonylamino. For example, one of $R_5$ and $R_6$ may represent nitro, cyano or alkylcarbonyl and the other may represent alkoxy, especially methoxy, amino, mono- or di-alkylamino or mono- or di-alkylcarbonylamino.

When $R_5$ or $R_6$ represents a substituted alkyl group, suitable optional substituents include one or more of halogen, hydroxy, alkoxy, thiol, nitro, cyano and alkylcarbonyl, especially halogen.

In one particular aspect, the present invention provides a compound of formula (I) or, where appropriate, a pharmaceutically acceptable salt thereof, or a pharmaceutically acceptable solvate thereof and wherein:

X represents oxygen or sulphur;

Y represents N or $N^+-O^-$ or a moiety $CR_6$ wherein $R_6$ is as defined below;

Z represents 0, $CH_2$ or $NR^o$;

$R^o$ represents hydrogen, $C_{1-6}$ alkyl or $C_{1-6}$ alkylcarbonyl

$R_1$ and $R_2$ independently represent hydrogen or $C_{1-6}$ alkyl; or R and $R_2$ together represent a $C_{2-7}$

polymethylene moiety;

$R_3$ is hydrogen; $C_{1-6}$ alkyl optionally substituted by halogen, hydroxy, $C_{1-6}$ alkoxy, $C_{1-6}$ alkoxycarbonyl, carboxy or amino optionally substituted by one or two independent $C_{1-6}$ alkyl groups; or $C_{2-6}$ alkenyl; amino optionally substituted by a $C_{1-6}$ alkyl or C2-6 alkenyl group or by a $C_{1-6}$ alkanoyl group optionally substituted by up to three halo atoms, by a phenyl group optionally substituted by $C_{1-6}$ alkyl, $C_{1-6}$ alkoxy or halogen; or aryl or heteroaryl, either being optionally substituted by one or more groups or atoms selected from the class of $C_{1-6}$ alkyl, $C_{1-6}$ alkoxy, hydroxy, halogen, trifluoromethyl, nitro, cyano, $C_{1-12}$ carboxylic acyl, or amino or aminocarbonyl optionally substituted by one or two $C_{1-6}$ alkyl groups; and

$R_4$ represents hydrogen or $C_{1-6}$ alkyl;

or $R_3$ and $R_4$ together represent $-CH_2-(CH_2)_n-Z^1-(CH_2)_m-$wherein m and n are 0 to 2 such that m + n is 1 or 2 and $Z^1$ is $CH_2$, O, S or NR wherein R is hydrogen, $C_{1-9}$ alkyl, $C_{2-7}$ alkanoyl, phenyl $C_{1-4}$- alkyl, naphthylcarbonyl, phenylcarbonyl or benzylcarbonyl optionally substituted in the phenyl or naphthyl ring by one or two of $C_{1-6}$ alkyl, $C_{1-6}$ alkoxy or halogen; or R is mono- or bi-cyclic- heteroarylcarbonyl;

when Y is N or $N^+-O^-$, $R_5$ is hydrogen or, when Y is $CR_6$, either one of $R_5$ and $R_6$ is hydrogen and the other is selected from the class of $C_{1-6}$ alkylcarbonyl, $C_{1-6}$ alkoxycarbonyl, $C_{1-6}$ alkylcarbonyloxy, $C_{1-6}$ alkylhydroxymethyl, nitro, cyano, chloro, trifluoromethyl, $C_{1-6}$ alkylsulphinyl, $C_{1-6}$ alkylsulphonyl, $C_{1-6}$ alkoxysulphinyl, $C_{1-6}$ alkoxysulphonyl, $C_{1-6}$ alkylcarbonylamino, $C_{1-6}$ alkoxycarbonylamino, $C_{1-6}$ alkyl-thiocarbonyl, $C_{1-6}$ alkoxy-thiocarbonyl, $C_{1-6}$ alkyl-thiocarbonyloxy, $C_{1-6}$ alkyl-thiomethyl, formyl or aminosulphinyl, aminosulphonyl or aminocarbonyl, the amino moiety being optionally substituted by one or two $C_{1-6}$ alkyl groups, or $C_{1-6}$ alkylsulphinylamino, $C_{1-6}$ alkylsulphonylamino $C_{1-6}$ alkoxysulphinylamino or $C_{1-6}$ alkoxysulphonylamino or ethenyl terminally substituted by $C_{1-6}$ alkylcarbonyl, nitro or cyano, or -C-($C_{1-6}$ alkyl)NOH or -C($C_{1-6}$ alkyl)$NNH_2$; or one of $R_5$ and $R_6$ is nitro, cyano or $C_{1-3}$ alkylcarbonyl and the other is methoxy or amino optionally substituted by one or two $C_{1-6}$ alkyl or by $C_{2-7}$ alkanoyl; or $R_5$ is hydrogen and $R_6$ is $C_{1-6}$ alkyl or $C_{3-8}$ cycloalkyl.

Preferably X is oxygen.

Preferably Y represents C-$R_6$ wherein $R_6$ is as defined in relation to formula (I).

Preferably, Z represents O.

Suitable $C_{1-6}$ alkyl groups or $C_{1-6}$ alkyl containing moieties may be selected from methyl, ethyl, n- and iso-propyl, n, iso-, sec- and tert-butyl. Suitable polymethylene groups include $C_3$, $C_4$, $C_5$, $C_6$ and $C_7$ polymethylene groups.

Preferably, $R_1$ and $R_2$ are both $C_{1-6}$ alkyl, and in particular $R_1$ and $R_2$ are both methyl.

Suitable aryl groups include phenyl and naphthyl, preferably phenyl.

Suitable heteroaryl groups include 5- or 6-membered monocyclic or 9- or 10-membered bicyclic heteroaryl groups, preferably 5- or 6-membered monocyclic heteroaryl groups.

Examples of 5- or 6-membered monocyclic or 9- or 10-membered bicyclic heteroaryl groups are those containing one, two or three heteroatoms selected from the class of oxygen, nitrogen and sulphur and which, in the case of there being more than one heteroatom, are the same or different.

Examples of 5- or 6-membered monocyclic heteroaryl moieties include furyl, thienyl, pyrrolyl, oxazolyl, thiazolyl, imidazolyl and thiadiazolyl, and pyridyl, pyridazyl, pyrimidyl, pyrazyl and triazyl. Preferred 5- or 6-membered heteroaryl groups include furyl, thienyl, pyrrolyl and pyridyl, in particular 2- and 3-furyl, 2- and 3-pyrrolyl 2- and 3-thienyl, and 2-, 3-and 4-pyridyl.

Suitable 9- or 10-membered bicyclic heteroaryl moieties include benzofuryl, benzothienyl, indolyl and indazolyl, quinolinyl and isoquinolinyl, and quinazolinyl. Preferred 9- or 10- membered bicyclic heteroaryl groups include 2- and 3-benzofuryl, 2- and 3-benzothienyl, and 2- and 3-indolyl, and 2- and 3-quinolinyl.

In any optionally substituted aryl or optionally substituted heteroaryl group, the preferred number of substituents is 1, 2, 3 or 4.

Preferred substituents for any substituted aryl or heteroaryl group include methyl, methoxy, hydroxy, chloro, nitro or cyano.

Preferably, when $R_3$ and $R_4$ together represent the moiety $-CH_2-(CH_2)_n-Z^1-(CH_2)_m-$as hereinbefore defined, the moiety $R_3$.N.CX.$R_4$ represents either pyrrolidonyl or piperidonyl.

When $Z^1$ is other than $CH_2$, m is often 0 or 1 and n is often 0 or 1.

Suitable examples of R when $Z^1$ is NR include hydrogen, methyl, ethyl, n- and iso-propyl, n-, sec- and tert-butyl, benzyl, phenylcarbonyl or benzylcarbonyl optionally substituted in the phenyl ring by methyl, methoxy, chloro or bromo; furylcarbonyl, thienylcarbonyl, pyrrolylcarbonyl or indolylcarbonyl.

Preferably R is hydrogen, methyl, n-butyl, acetyl, benzyl, benzylcarbonyl, phenylcarbonyl or furylcarbonyl.

Most preferably R is methyl.

When one of $R_5$ and $R_6$ represents nitro, cyano or $C_{1-3}$ alkylcarbonyl the other is, preferably, amino

optionally substituted by one or two $C_{1-6}$ alkyl or by $C_{2-7}$ alkanoyl. In particular, when one of $R_5$ and $R_6$ is nitro, cyano or $C_{1-3}$ alkylcarbonyl, the other is amino, methylamino, dimethylamino or acetylamino. Most preferably, one of $R_5$ and $R_6$ is nitro or cyano, and the other is amino.

When one of $R_5$ and $R_6$ is nitro, cyano or $C_{1-3}$ alkylcarbonyl, it is preferred that $R_6$ is nitro, cyano or $C_{1-3}$ alkylcarbonyl.

When one of $R_5$ and $R_6$ represents hydrogen, it is preferred that $R_5$ is hydrogen.

$R_6$ is preferably selected from the class of alkoxycarbonyl, nitro, cyano or fluoroalkyl, especially when $R_5$ is hydrogen.

In particular, $R_6$ may be acetyl, nitro, cyano, trifluoromethyl or pentafluoroethyl.

It is generally preferred, however, that $R_5$ is hydrogen.

It is generally preferred, however, that $R_6$ is cyano.

Thus in one preferred aspect $R_5$ is hydrogen and $R_6$ is cyano.

The alkyl groups or alkyl moieties of alkyl-containing groups for $R_5$ or $R_6$ are, preferably, methyl or ethyl.

When used herein the term "halogen" refers to fluorine, chlorine, bromine and iodine; preferably chlorine or fluorine.

Suitably alkyl groups, or alkyl groups forming part of other groups such as in the alkoxy group, are $C_{1-12}$ alkyl groups especially $C_{1-6}$ alkyl groups e.g. methyl, ethyl, n-propyl, iso-propyl, n-butyl, isobutyl or tert-butyl groups.

Suitably alkenyl groups are $C_{2-12}$ groups especially $C_{2-6}$ alkenyl groups.

Suitable alkynyl groups are $C_{2-12}$ alkynyl groups especially $C_{2-6}$ alkynyl groups.

Suitable polymethylene groups include $C_3$, $C_4$, $C_5$, $C_6$ and $C_7$ polymethylene groups.

Suitable acyloxy groups include alkylcarbonyloxy groups wherein the alkyl group is as defined above.

When used herein the term "fluoroalkyl" or "fluoroalkoxy" includes alkyl groups as defined above when substituted by one or more fluorine atoms, particular examples being trifluoromethyl and pentafluoroethyl.

Suitable pharmaceutically acceptable salts of the compounds of formula (I) include acid addition salts and salts of carboxy groups.

Examples of pharmaceutically acceptable acid addition salts of the compounds of formula (I) includes acid addition salts of optionally substituted amino groups, such as the hydrochloride and hydrobromide salts. Such a salifiable group may form part of an $R_5$ group. It will also be appreciated that when Y in the compound of formula (I) represents N, then the resulting pyridine moiety may yield acid addition salts, such as the hydrochloride or hydrobromide salts.

Examples of pharmaceutically acceptable salts of carboxy groups include metal salts, such as alkali metal salts, or optionally substituted ammonium salts.

Examples of esters of carboxy groups are pharmaceutically acceptable esters such as $C_{1-6}$ alkyl esters.

Examples of amides of carboxyl groups include pharmaceutically acceptable amides such as amides of formula $-CO.NR^sR^t$ wherein $R^s$ and $R^t$ each independently represent hydrogen or $C_{1-6}$ alkyl.

The compounds of formula (I) may also exist in the form of solvates, preferably hydrates, and the invention extends to such solvates.

The compounds of formula (I), may exist in the form of optical isomers. The asterisked carbon atoms in formula (I) are chiral carbon atoms. Chirality arises when $R_1$ and $R_2$ are different. Chirality may also arise in those compounds of formula (I) wherein $R_3$ and $R_4$ together represent a linking group $-A^1-A^2-$, as the said linking group may possess up to 4 chiral carbon atoms. The present invention extends to all optical isomers of the compounds of formula (I) whether in the form of single isomers or of mixtures thereof, such as racemates.

The compounds of formula (I) may also exist in geometrical isomeric forms all of which are encompassed by the present invention.

Particular examples of compounds of formula (I) include the compounds prepared in the Examples hereinafter.

The present invention also provides a process for the preparation of a compound of formula (I) or, where appropriate a pharmaceutically acceptable salt thereof, or a pharmaceutically acceptable solvate thereof, which process comprises deprotecting a compound of formula (II):

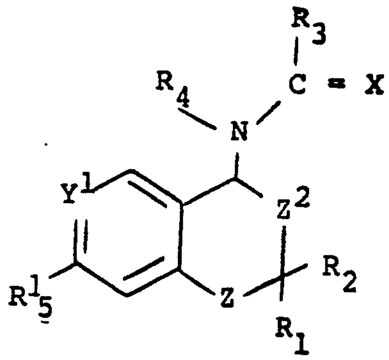

(II)

wherein $R_1$, $R_2$, $R_3$, $R_4$, X and Z are as defined in relation to formula (I), $R^1_5$ represents $R_5$ or a group or atom convertible thereto, $Y^1$ represents Y or a group convertible thereto and $Z^2$ represents a protected keto group; and thereafter, if required, carrying out one or more of the following optional steps:

(i) converting a compound of formula (I) into a further compound of formula (I):

(ii) converting $Y^1$ to Y and/or $R^1_5$ to $R_5$;

(iii) forming a pharmaceutically acceptable salt of the compound of formula (I) and/or a pharmaceutically acceptable solvate thereof.

Suitably, $Z^2$ represents a ketal, favourably a cyclic ketal, for example a 3,3-propylenedioxy group.

The deprotection of the compound of formula (I) is suitably carried out under conditions appropriate to the nature of the group $Z^2$, for example when $Z^2$ represents a ketal, such as a cyclic ketal, the compound of formula (III) may be deprotected using mild acid conditions such as is provided by aqueous trifluoroacetic acid at 50 °C.

A compound of formula (II) may be prepared by reacting a compound of formula (III):

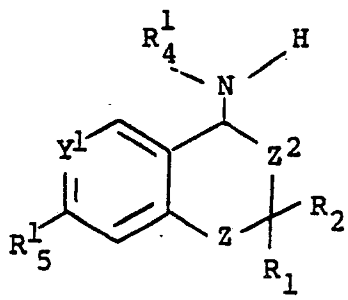

(III)

wherein $R_1$, $R_2$ $R^1_5$, $Y^1$, Z and $Z^2$ are as defined hereinbefore and $R^1_4$ is hydrogen or alkyl:

i) for compounds of formula (I) other than those wherein the moiety $R_4$.N.CX.$R_3$ represents a substituted or unsubstituted 2-pyridonyl or 2-thiopyridonyl group:

a) with an acylating agent of formula (IV):

$R_7$-CO-$L_1$ (IV)

wherein $L_1$ is a leaving group, and $R_7$ is alkyl optionally substituted by halogen, hydroxy, alkoxy, alkoxycarbonyl, carboxy or amino optionally substituted as hereinbefore defined for $R_3$, alkenyl or optionally substituted aryl or heteroaryl as hereinbefore defined for $R_3$, or a group convertible to $R_3$ as hereinbefore defined; or

b) when $R^1_4$ represents hydrogen, with an acylating agent of formula (V):

$X^1$-$R_8$-CO-$L_1$ (V)

wherein $L_1$ is as defined above, $R_8$ represents a linking chain of formula -$A^1$-$A^2$- or a protected form thereof and $X^1$ represents a leaving group, and thereafter cyclising the resulting compound; or

c) with a compound of formula (VI)

$X^2$=C=N.$R_9$ (VI)

wherein $R_9$ is hydrogen, alkyl, alkenyl, alkanoyl optionally substituted by up to three halo atoms, or phenyl optionally substituted by alkyl, alkoxy or halogen; and $X^2$ is oxygen or sulphur, and thereafter when $R_9$ is hydrogen, optionally converting $R_9$ into another $R_9$; or

(ii) for compounds of formula (I) wherein the moiety $R_4$.N.CX.$R_3$ represents a 2-pyridonyl or 2-thiopyridonyl group, by reacting a compound of the abovedefined formula (III) with a 2-pyrone or 2-thiopyrone of formula (VII):

$R_4$-O-CX-$R_3$ (VII)

wherein $R_3$, $R_4$ and CX represent the corresponding variables of moiety $R_4$.N.CX.$R_3$; and thereafter if required, carrying out one or more of the following optional steps:

(i) converting a compound of formula (I) into a further compound of formula (I);

(ii) converting $Y^1$ to Y;

(iii) converting $R_5{}^1$ to $R_5$;

(iv) forming a pharmaceutically acceptable salt of the compound of formula (I);

(v) forming a pharmaceutically acceptable solvate of the compound of formula (I) or a pharmaceutically acceptable salt thereof.

Preferably, $R_8$ in the compound of formula (V) represents $(CH_2)_r$ wherein r represents an integer 3 or 4.

In the process variant a), that is the acylation of a compound of formula (III) with an acylating agent of formula (IV), the leaving group $L_1$ is a group that is displaceable by a primary or secondary amino nucleophile. Examples of such a group include $C_{1-4}$ alkanoyloxy, and halogen, such as chloro and bromo. When the leaving group $L_1$ is either of these examples, the acylating agent of formula (IV) is either an acid anhydride or an acid halide. When it is an acid anhydride, it may be a mixed or simple anhydride. If it is a mixed anhydride, it may be prepared in situ from a carboxylic acid and an acid halide, although this is less preferred than using the halide itself. When L1 is hydroxy, conventional coupling methods using dicyclohexylcarbodiimide are suitable.

In process variant a), when $R_3$ in the desired compound of formula (I) is an optionally substituted amino-substituted alkyl group as hereinbefore defined, it is preferred that $R_7$ is a group convertible to the $R_3$ substituted alkyl group as hereinbefore defined, in particular that it is alkyl substituted by halo, especially bromo. The $R_7$ halo substituent in the resultant compound of process variant a) may be converted to an $R_3$ substituent which is amino optionally substituted as hereinbefore defined by a conventional amination reaction with ammonia or a corresponding alkyl- or dialkylamine.

When the acylating agent of formula (IV) is an acid anhydride, the acylation of the compound of formula III) may be carried out in the presence of an acid acceptor, such as sodium acetate, optionally using the anhydride as the solvent.

When the acylating agent of formula (IV) is an acid halide, the acylation of the compound of formula (III) is, preferably, carried out in a non-aqueous medium, such as dichloromethane, in the presence of an acid acceptor, such as triethylamine, trimethylamine, pyridine, picoline or calcium, potassium or sodium carbonate.

In the process variant b), the leaving group $X^1$ in the compound of formula (V) is a group that is displaceable by a secondary amino nucleophile adjacent to a carbonyl function. A preferred example is chloro.

The cyclisation reaction in process variant b) is preferably carried out in an aprotic solvent such as dimethylformamide at any temperature which provides a suitable rate of formation of the product, suitably at an elevated temperature, for example 60°C, preferably in the presence of a base, such as potassium carbonate and preferably in the presence of a catalyst, such as sodium iodide.

In process variant c), when $R_9$ in a compound of formula (VI) is alkyl, alkanoyl optionally substituted as hereinbefore defined, or phenyl optionally substituted as hereinbefore defined, the reaction between the compounds of formulae (III) and (VI) is, preferably, carried out in a solvent, such as methylene chloride, at below room temperature, in particular below 10°C.

When $R_9$ is hydrogen, the reaction between the compounds of formulae (III) and (VI) is, preferably, carried out using a corresponding alkali metal cyanate or thiocyanate, for example that of sodium or potassium, in an optionally methanolic aqueous medium acidified with a mineral acid, such as dilute hydrochloride acid. A slightly elevated temperature such as 50 to 90°C is apt.

In process variant ii), the reaction between the compounds of the above defined formulae (III) and (VII) may be carried out in any suitable solvent, such as dioxane, at any convenient temperature providing a suitable rate of formation of the required product, for example a temperature within the range of from 30 to 100°C.

Suitable conversions of a compound of formula (I) to a further compound of formula (I) include thiating the $R_4$.N.CO.$R_3$ group in the compound of formula (I) to give a group $R_4$.N.CS.$R_3$, that is wherein X represents sulphur.

The thiation of the $R_4$.N.CO.$R_3$ group in a compound of formula (I) to give another compound of formula (I), wherein X is sulphur, is, preferably, carried out with conventional thiation agents, such as hydrogen sulphide, phosphorus pentasulphide and Lawesson's reagent (p-methoxyphenylthiophosphine sulphide dimer). The use of hydrogen sulphide and phosphorus pentasulphide may lead to side-reactions and,

therefore, the use of Lawesson's reagent is preferred.

The thiation reaction conditions are conventional for the thiation agent employed. For example, the use of hydrogen sulphide is, preferably, acid catalysed by, for example, hydrogen chloride in a polar solvent, such as acetic acid or ethanol. The preferred use of Lawesson's reagent is, preferably, carried out under reflux in a dry solvent, such as toluene or methylene chloride.

The required conversion of $Y^1$ to Y or $R_5{}^1$ to $R_5$ may be carried out using the appropriate conventional chemical procedure.

The optional formation of a pharmaceutically acceptable salt, when the resulting compound of formula (I) contains a salifiable group, may be carried out conventionally. Similarly, pharmaceutically acceptable solvates, for example hydrates, may be prepared using any convenient conventional procedure.

A compound of formula (III) may be prepared by reacting a compound of formula (VIII):

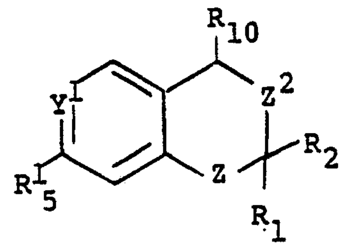

(VIII)

wherein $R_1$, $R_2$, $R^1_5$, $Y^1$, Z and $Z^2$ are as defined above and $R_{10}$ is a group convertible to a group $R^1_4$. N .H

wherein $R^1_4$ is as defined above, with a reagent capable of converting R10 into a group $R^1_4$. N .H.

When $R^1_4$ represents hydrogen, $R_{10}$ may conveniently represent $-N_3$. A suitable reagent for converting $-N_3$ into $-NH_2$ is a reducing agent, for example activated zinc dust in a solvent such as methanol or tetrahydrofuran or a mixture thereof. The reduction may be carried out at any temperature which provides a suitable rate of formation of the product, such as at ambient temperature and preferably in the presence of a catalyst such as ammonium chloride.

When $R^1_4$ represents alkyl, $R_{10}$ may also represent $-N_3$, but in this instance the amino group produced suitably by reduction of the azido group, is subsequently alkylated using conventional alkylation methods, such as the use of a alkyl halide in the presence of a base or the use of the appropriate aldehyde followed by reduction with an appropriate reducing agent.

The nature of the substrate to the compound of formula (VIII) will of course depend upon the nature of the group $R_{10}$ but when $R_{10}$ represents $-N_3$ the compound of formula (VIII) is conveniently prepared by reacting a compound of formula (IX):

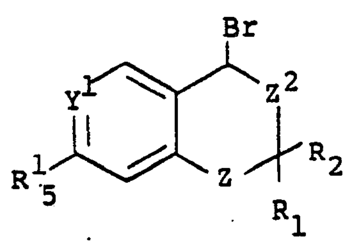

(IX)

wherein $R_1$, $R_2$, $R^1_5$, $Y^1$, Z and $Z^2$ are as defined above, with a source of azide ions.

A suitable source of azide ions is an alkali metal azide, such as sodium azide.

The reaction between the compound of formula (IX) and the source of azide ions may be carried out under the conventional conditions dictated by the nature of the source of azide ions, for example when the source is an alkali metal azide the reaction may conveniently be carried out in a solvent such as aqueous-dioxan, conveniently at an elevated temperature such as the reflux temperature of the solvent.

A compound of formula (IX) may conveniently be prepared by brominating a compound of formula (X):

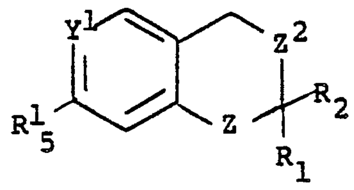

(X)

wherein $R_1$, $R_2$, $R_5^1$, $Y^1$, Z and $Z^2$ are as defined above.

The bromination of the compound of formula (X) is suitably carried out using N-bromosuccinimide in an inert solvent, such as carbon tetrachloride, at an ambient to elevated temperature, conveniently the reflux temperature of the solvent, preferably in the presence of ultraviolet light and preferably in the presence of a suitable catalyst such as azobisisobutyronitrile (AIBN).

A compound of formula (X) may conveniently be prepared by treating a compound of formula (XI):

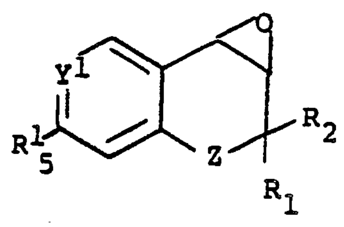

(XI)

wherein $R_1$, $R_2$, $R_5^1$, $Y^1$ and Z are as defined above, with an appropriate Lewis acid, and thereafter, if required, protecting the keto group so formed.

An appropriate Lewis acid is a mild Lewis acid, such as magnesium bromide.

Suitably, the keto group may be protected as a ketal, such as a cyclic ketal, for example a propylene-1,3-dioxyl group, by reacting the keto group with an appropriate alcohol, such as a diol, for example propane-1,3-diol, in a solvent such as benzene, preferably in the presence of an acid catalyst, such as p-toluenesulphonic acid.

The reaction between the compound of formula (XI) and the Lewis acid may be carried out in any suitable inert solvent, such as toluene, at any suitable temperature, conveniently at an elevated temperature such as in the range of from 40° to 110° C, for example at 80° C.

The compounds of formula (X) are known compounds or they may be prepared according to analogous procedures used to prepare known compounds, for example those disclosed in the abovementioned European applications.

As mentioned previously, some of the compounds of formula (I) may exist in optically active form, and the processes of the present invention produce mixtures of such forms. The individual enantiomers may be separated by conventional methods.

It is preferred that the compounds of formula (I) are isolated in substantially pure form.

The intermediates of formulae (II), (III), (VIII), (IX) and (X) are believed to be novel and accordingly they form part of the present invention.

The compounds of formulae (IV), (V), (VI) and (VII) are known commercially available compounds and may be prepared using conventional procedures, for example those disclosed in 'Advanced Organic Chemistry', J. March, New York, Wiley, Interscience, 3rd Ed., 1985.

The compounds of formula (I), the pharmaceutically acceptable salts thereof, or the pharmaceutically acceptable solvates thereof, have been found to have bronchodilator activity and/or blood-pressure lowering activity. They are therefore useful in the treatment of respiratory tract disorders, such as reversible airways obstruction, diverticular disease and asthma and also hypertension. They may also be of potential use in the treatment of other disorders hereinbefore described.

The present invention accordingly provides a pharmaceutical composition which comprises a compound of formula (I), or a pharmaceutically acceptable salt thereof or a pharmaceutically acceptable solvate thereof, and a pharmaceutically acceptable carrier.

The compositions are preferably adapted for oral administration. However, they may be adapted for other modes of administration, for example in the form of a spray, aerosol or other conventional method for inhalation, for treating respiratory tract disorders; or parenteral administration for patients suffering from heart failure. Other alternative modes of administration include sublingual or transdermal administration.

The compositions may be in the form of tablets, capsules, powders, granules, lozenges, suppositories, reconstitutable powders, or liquid preparations, such as oral or sterile parenteral solutions or suspensions.

In order to obtain consistency of administration it is preferred that a composition of the invention is in the form of a unit dose.

Unit dose presentation forms for oral administration may be tablets and capsules and may contain conventional excipients such as binding agents, for example syrup, acacia, gelatin, sorbitol, tragacanth, or polyvinylpyrrolidone; fillers, for example lactose, sugar, maize-starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulphate.

The solid oral compositions may be prepared by conventional methods of blending, filling or tabletting. Repeated blending operations may be used to distribute the active agent throughout those compositions employing large quantities of fillers. Such operations are of course conventional in the art. The tablets may be coated according to methods well known in normal pharmaceutical practice, in particular with an enteric coating.

Oral liquid preparations may be in the form of, for example, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethylcellulose, aluminium stearate gel, hydrogenated edible fats; emulsifying agents, for example lecithin, sorbitan monooleate, or acacia; non-aqueous vehicles (which may include edible oils), for example almond oil, fractionated coconut oil, oily esters such as esters of glycerine, propylene glycol, or ethyl alcohol; preservatives, for example methyl or propyl p-hydroxybenzoate or sorbic acid; and if desired conventional flavouring or colouring agents.

For parenteral administration, fluid unit dosage forms are prepared utilizing the compound and a sterile vehicle, and, depending on the concentration used, can be either suspended or dissolved in the vehicle. In preparing solutions the compound can be dissolved in water for injection and filter sterilized before filling into a suitable vial or ampoule and sealing. Advantageously, adjuvants such as a local anaesthetic, a preservative and buffering agents can be dissolved in the vehicle. To enhance the stability, the composition can be frozen after filling into the vial and the water removed under vacuum. Parenteral suspensions are prepared in substantially the same manner, except that the compound is suspended in the vehicle instead of being dissolved, and sterilization cannot be accomplished by filtration. The compound can be sterilized by exposure to ethylene oxide before suspending in the sterile vehicle. Advantageously, a surfactant or wetting agent is included in the composition to facilitate uniform distribution of the compound.

Compositions of this invention may also suitably be presented for administration to the respiratory tract as a snuff or an aerosol or solution for a nebulizer, or as a microfine powder for insufflation, alone or in combination with an inert carrier such as lactose. In such a case the particles of active compound suitably have diameters of less than 50 microns, preferably less than 10 microns. Where appropriate, small amounts of other anti-asthmatics and bronchodilators, for example sympathomimetic amines such as isoprenaline, isoetharine, salbutamol, phenylephrine and ephedrine; xanthine derivatives such as theophylline and aminophylline and corticosteroids such as prednisolone and adrenal stimulants such as ACTH may be included.

The compositions may contain from 0.1% to 99% by weight, preferably from 10-60% by weight, of the active material, depending on the method of administration.

The present invention further provides a method of treatment of respiratory tract disorders or hypertension in mammals including man, which comprises administering to the suffering mammal an effective amount of a compound of formula (I), or a pharmaceutically acceptable salt thereof or a pharmaceutically acceptable solvate thereof.

An effective amount will depend on the relative efficacy of the compounds of the present invention, the severity of the respiratory tract disorder or hypertension being treated and the weight of the sufferer. However, a unit dose form of a composition of the invention may contain from 0.01 to 100mg of a compound of the invention (0.01 to 10mg via inhalation) and more usually from 0.1 to 50mg, for example 0.5 to 25mg such as 1, 2, 5, 10, 15 or 20mg. Such compositions may be administered from 1 to 6 times a day, more usually from 2 to 4 times a day, in a manner such that the daily dose is from 0.02 to 200mg for a 70 kg human adult and more particularly from 0.05to 100mg.

No toxicological effects are indicated at the aforementioned dosage ranges.

The present invention further provides a compound of formula (I) or a pharmaceutically acceptable salt thereof, or a pharmaceutically acceptable solvate thereof, for use in the treatment of respiratory tract disorders or hypertension.

The present invention further provides the use of a compound of formula (I), or a pharmaceutically acceptable salt thereof, or a pharmaceutically acceptable solvate thereof for the manufacture of a medicament for the treatment of respiratory tract disorders or hypertension.

The following Procedures relate to the preparation of intermediates and the following Examples relate to the preparation of compounds of formula (I).

EXAMPLE 1

6-Cyano-3,4-dihydro-2,2 dimethyl-4-(2-oxopyrrolidin-1-yl)-2H-1-benzopyran-3-one

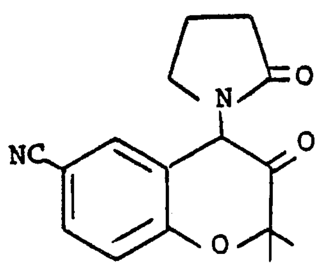

A solution of 6-cyano-3,4-dihydro-2,2-dimethyl-4-(2-oxopyrrolidin-1-yl)-3,3-propylenedioxy-2H-1-benzopyran (0.992g, 3.06 mmol) in trifluoroacetic acid (80ml) and water (25ml) was heated at 50°C for 16 hours. The reaction mixture was cooled, concentrated under vacuum and the residue partitioned between dichloromethane and water. The organic phase was washed with brine, dried over magnesium sulphate, filtered then concentrated to give a pink solid 930mg).. Purification by chromatography ($SiO_2$/EtOAC) provided the title compound as a white solid (780mg, 90%), m.p. 162 - 163°C.
IR (KBr): 2620, 2230, 1745, 1700 - 1600cm$^{-1}$.
$^1$Hnmr (DMS0-$d_6$) (mainly the enol form): δ 1.45 (6H,s), 2.14 (2H, m,J=7.5 Hz), 2.38 (2H,m), 3.43 (2H,t,J=7 Hz), 6.89 (1H, d,J=8 Hz), 7.10 (1H,d, J=2 Hz), 7.41 (1H, dd, 8 and 2 Hz), 10.18 (1H,m).
$^1$Hnmr ($CDCl_3$) (mainly the ketone form): δ 1.4 (3H,s), 1.6 (3H,s), 2.2 (2H,m) 2.6 (2H, t, J=8 Hz), 3.18 (1H,td,J=5 and 9 Hz), 3.36 (1H, td, J=8 and 8 Hz), 5.92 (1H,s), 7.1 (1H,d,J=8 Hz), 7.32 (1H,d,J=1.5 Hz), 7.59 (1H,dd,J=8 and 1.5 Hz).
Anal: Found C, 66.74; H, 5.71; N, 9.47%. Requires C,67.59; H, 5.67; N, 9.85%
Accurate Mass Measurement: observed mass 284.1155, theoretical mass: 284.1161.

PREPARATION 1

6 Cyano-3,4-dihydro-2,2-dimethyl-2H-1-benzopyran-3-one

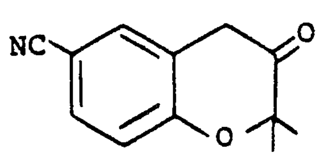

A solution of 6-cyano-3,4-dihydro-2,2-dimethyl-3,4-epoxy-2H-1-benzopyran (10.73g, 53.38mmol) in dry toluene (160ml) was treated with magnesium bromide etherate (13.78g, 53.38 mmol) at 80°C for two hours. The reaction mixture was cooled, treated with hydrochloric acid (2M) and extracted with dichloromethane. The organic phase was dried over magnesium sulphate, filtered and concentrated to give a brown solid (11g). Purification by chromatography ($SiO_2$- $CHCl_3$) yielded 6-cyano-3,4-dihydro-2,2-dimethyl-2H-1-benzopyran-3-one as a white solid (6.15g, 61%), m.p. 98 - 99°C.

IR ($CH_2Cl_2$): 2220 and 1730 $cm^{-1}$.
$^1$Hnmr ($CDCl_3$): δ 1.5 (6H,s), 3.6 (2H,s), 7.05 (1H,d,J=8 Hz), 7.4 (1H,d,J=2 Hz), 7.55 (1H,dd,J=8 and 2 Hz).
Anal : Found C, 71.46; H,5.49; N, 6.96. $C_{12}H_{11}NO_2$ requires C, 71.63; H,5.51, N, 6.96%.

PREPARATION 2

6-Cyano-3,4-dihydro-2,2-dimethyl-3,3-propylenedioxy-2H-1-benzopyran

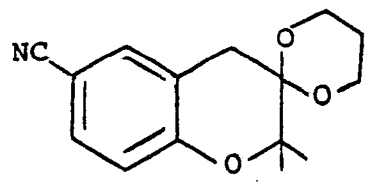

A solution of 6-cyano-3,4-dihydro-2,2-dimethyl-2H-1-benzopyran-3-one (4.98g, 24.7mmol) in benzene (100ml) was treated with propane-1,3-diol (9.4g, 0.123mol) and p-toluenesulphonic acid (0.47g, 2.47 mmol) in a Dean-stark apparatus. The reaction mixture was heated to 80° C for four hours then allowed to cool and poured into water. The aqueous phase was extracted with ethyl acetate. The combined organic extracts were washed with aqueous sodium bicarbonate, dried over magnesium sulphate, filtered and concentrated to give an orange oil (5.87g). Purification by chromatography ($SiO_2$, 1:1 $CH_2Cl_2$ - hexane) provided 6-cyano-3,4-dihydro-2,2-dimethyl-3,3-propylenedioxy-2H-1-benzopyran as a white solid (3.42g, 54%), m.p. 92-93° C.
$^1$Hnmr ($CDCl_3$): δ 1.37 (6H,s), 1.45 (1H,m), 2.1 (1H,m), 3.25 (2H,s), 3.95 (4H,m), 6.87 (1H,d,J=8 Hz), 7.38 (1H,s), 7.40 (1H,J=8 Hz).
Anal:Found C, 69.51; H, 6.68; N,5.47. $C_{15}H_{17}NO_3$ requires C, 69.41; H, 6.62; N, 5.40%.

PREPARATION 3

4-Bromo-6-cyano-3,4-dihydro-2,2-dimethyl-3,3-propylenedioxy-2H-1-benzopyran

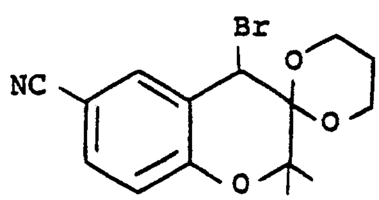

A solution of 6-cyano-3,4-dihydro-2,2-dimethyl-3,3-propylenedioxy-2H-1-benzopyran (1.3g, 5mmol) in carbon tetrachloride (20ml) was treated with N-bromosuccinimide (1.25g, 7mmol) and AIBN (2 mg). The reaction mixture was heated under reflux and illuminated by a tungsten lamp for three hours then dried over magnesium sulphate, filtered and concentrated to give an orange oil (2.32g). Purification by chromatography ($SiO_2$, 3:2 $CH_2Cl_2$-hexane)provided 4-bromo-6-cyano-3,4-dihydro-2,2-dimethyl-3,3-propylenedioxy-2H-1-benzopyran as a white solid (0.704g, 42%).
$^1$Hnmr ($CDCl_3$): δ 1.52 (6H s), 1.52 (1H,m), 2.1 (1H,m), 3.9-4.15 (4H,m), 5.8 (1H,s), 6.89 (1H,d,J=8.5 Hz), 7.43 (1H,dd J=8.5 and 2 Hz), 7.77 (1H,d, J=2 Hz).
Anal: Found C, 52.87, H, 4.88; N, 4.09. $C_{15}H_{16}NO_3Br$ requires C, 53.27; H, 4.77; N, 4.14%.

PREPARATION 4

4-Azido-6-cyano-3,4-dihydro-2,2-dimethyl-3,3-propylenedioxy-2H-1-benzopyran

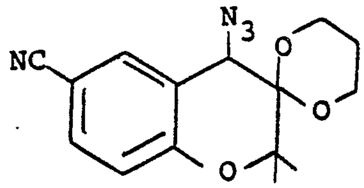

A solution of 4-bromo-6-cyano-3,4-dihydro-2,2-dimethyl-3,3-propylenedioxy-2H-1-benzopyran (0.940g, 2.78mmol) in dioxan-water (7ml-1.5 ml) was treated with sodium azide (0.624g 9.6 mmol). The reaction mixture was refluxed for 64 hours then cooled and concentrated. The residue was extracted with dichloromethane and washed with water. The organic phase was dried over magnesium sulphate, filtered and concentrated to give a yellow oil. Purification by chromatography ($SiO_2$-$CH_2C_{12}$) provided 4-azido-6-cyano-3,4-dihydro-2,2-dimethyl-3,3-propylenedioxy-2H-1-benzopyran as a white solid (0.51g, 61%), m.p. 169 -170° C.
IR (Nujol): 2200, 2085 $cm^{-1}$.
$^1$Hnmr ($CDCl_3$): $\delta$ 1.41 (3H,s), 1.47 (3H,s), 1.6 (1H,m), 2.1 (1H, m),4.05 (4H,m), 5.13 (1H,s), 6.92 (1H,d, J=8.5Hz), 7.50 (1H,dd, J=8.5 and 2 Hz), 7.60 (1H,d, J= 2 Hz).
Anal: Found C,60.14; H,5.45; N, 18.07. $C_{15}H_{16}N_4O_3$ requires C, 59.98; H,5,37; N, 18.66%.

PREPARATION 5

4-Amino-6-cyano-3,4-dihydro-2,2-dimethyl-3,3-propylenedioxy-2H-1-benzopyran

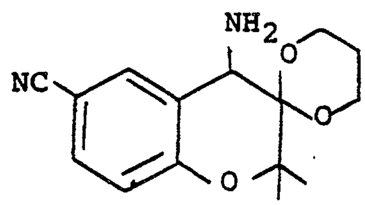

A solution of 4-azido-6-cyano-3,4-dihydro-2,2-dimethyl-3,3-propylenedioxy-2H-1-benzopyran (480 mg, 1.6 mmol) in methanol-tetrahydrofuran (2:1, 9 ml) was treated with activated zinc dust (465 mg 7.12 mmol) and ammonium chloride (704 mg, 13.2mmol). The reaction mixture was stirred at room temperature for 17 hours then filtered through a pad of celite and the filtrate washed with water. The organic phase was dried over magnesium sulphate, filtered and concentrated to give 4-amino-6-cyano-3,4-dihydro-2,2-dimethyl-3,3-propylenedioxy-2H-1-benzopyran as a colourless oil, (0.5g). This material was used without further purification.
IR (Nujol): 3400, 3340 and 2220 $cm^{-1}$.
$^1$Hnmr ($CDCl_3$): $\delta$ 1.42 (3H,s), 1.5 (3H,s), 1.3-2.2 (4H,m) 3.85-4.25 (4H,m), 4.47 (1H,s), 6.9 (1H,d,J=8 Hz), 7.5 (1H,dd, 8 and 2 Hz), 7.8 (1H,d, 2Hz).

PREPARATION 6

4-(4-Chlorobutyrylamino)-6-cyano-3,4-dihydro-2,2-dimethyl-3,3-propylenedioxy-2H-1-benzopyran.

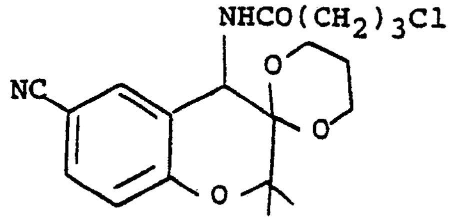

A solution of crude 4-amino-6-cyano-3,4-dihydro-2,2-dimethyl-3,3-propylenedioxy-2H-1-benzopyran (440mg, 1.6mmol) in dichloromethane (10ml) was treated with triethylamine (0.236ml, 1.6mmol) and chlorobutyryl chloride (0.185 ml, 1.6 mmol). The reaction mixture was stirred at room temperature for two hours then washed with water. The organic phase was dried over magnesium sulphate, filtered and concentrated to give 4-(4-chlorobutyrylamino)-6-cyano-3,4-dihydro-2,2-dimethyl-3,3-propylenedioxy-2H-1-benzopyran as a white foam (0.6g). This material was used without further purification.
IR (nujol): 3450, 2220 and 1680 $cm^{-1}$
$^1$Hnmr($CDCl_3$): δ 1.4 (3H,s), 1.45 (3H,s), 2.0-1.5 (2H,m), 2.1 (2H,m), 3.7-4.2 (4H,m),4.5 (1H,s),6.8 (1H,d,J=9 Hz), 7.4 (1H,dd,J=9 and 2 Hz), 7.78 (1H,d,J=2 Hz).

PREPARATION 7

6-Cyano-3,4-dihydro-2,2-dimethyl-4-(2-oxopyrrolidin-1-yl)-3,3-propylenedioxy-2H-1-benzopyran

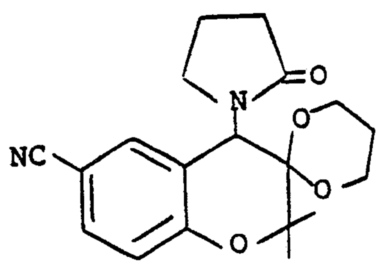

A solution of 4-(4-chlorobutyrylamino)-6-cyano-3,4-dihydro-2,2-dimethyl-3,3-propylenedioxy-2H-1-benzopyran (1.84g, 4.86mmol) in dimethylformamide (400ml) was treated with potassium iodide (3.06g, 18.42mmol) and potassium carbonate (35.69g, 214.82 mmol). The reaction mixture was stirred at 60° C for 20 hours then cooled and concentrated. The residue was partitioned between dichloromethane and water. The organic phase was dried over magnesium sulphate, filtered and concentrated to give an orange oil (1.94g). Purification by chromatography ($SiO_2$- EtOAC) yielded 6-cyano-3,4-dihydro-2,2-dimethyl-4-(2-oxopyrrolidin-1-yl) -3,3-propylenedioxy-2H-1-benzopyran as a white solid (1.125g, 68%).
IR ($CH_2Cl_2$): 2230 and 1675 $cm^{-1}$.
$^1$H nmr ($CDCl_3$): δ 1.38 (3H,s), 1.47 (3H,s), 1.5 (2H,m) 2.05 (2H,m),2.55 (2H,m), 3.15 (1H,m), 3.55 (1H,m), 3.8-4.3 (4H,m), 5.78 (1H,s), 6.94 (1H,d,J=9 Hz), 7.3 (1H,d, J= 2 Hz), 7.5 (1H,dd,J = 9 and 2 Hz).

Pharmacological Data

1. Bronchodilator Activity

(a) Bronchodilation in vitro; guinea pig tracheal spiral preparations.

Male guinea pigs (300-600g) were stunned by a blow to the head and bled from the carotid artery. The

trachea was exposed, dissected free of connective tissue, and transferred to oxygenated krebs solution at 37° C. Next, spirals (2 per trachea) were prepared by cutting the whole trachea spirally along its longitudinal axis and then dividing this spiral lengthwise. Each preparation was mounted, using silk thread, in a 10ml organ bath filled with krebs solution at 37° C and bubbled with 5% $CO_2$ with $O_2$. The resting tension of the preparations was set at 2g and changes in muscle tension were monitored isometrically by means of a UFI (2oz) force and displacement transducer (Ormed Ltd) connected to a Linseis pen recorder. All preparations were allowed to equilibrate for 60 minutes. During this equilibration period the preparations were washed by upward displacement at 15 minute intervals and, if necessary, the resting tension was readjusted to 2g using a mechanical micromanipulator system.

Once a steady resting tension had been obtained, the preparations were dosed cumulatively with the test compound ($10^{-8}$-$2x10^{-5}$M), and finally a maximum relaxation achieved by addition of $10^{-3}$M isoprenaline. The fall in tension evoked by the test compound was expressed as a percentage of the total relaxation evoked in the presence of $10^{-3}$M isoprenaline.

Appropriate concentration-relaxation curves were then constructed and values for potency ($IC_{50}$) and intrinsic activity (I.A.) were obtained.

The composition of Krebs solution is: sodium chloride 118.07mM, sodium hydrogen carbonate 26.19mM, potassium chloride 4.68mM, potassium orthophosphate 1.18mM, magnesium sulphate septahydrate 1.8mM and calcium chloride 2.52mM;pH ca. 7.45.

| Results | | |
|---|---|---|
| Example No . | In vitro $IC_{50}$ (M) | Intrinsic Activity |
| 1 | $3.55 \times 10^{-6}$ | 0.89 |

2. Antihypertensive Activity

Blood Pressure Lowering Activity

Systolic blood pressures were recorded by a modification of the tail cuff method described by I.M. Claxton, M.G. Palfreyman, R.H. Poyser, R.L. Whiting, European Journal of Pharmacology, 37, 179 (1976). A W+W BP recorder, model 8005 was used to display pulses.Prior to all measurements rats were placed in a heated environment (33.5 ± 0.5° C) before transfer to a restraining cage. Each determination of blood pressure was the mean of at least 6 readings. Spontaneously hypertensive rats (ages 12-18 weeks) with systolic blood pressures >180 mmHg were considered hypertensive.

| Results | | |
|---|---|---|
| Compound of Example 1 | Time, post (h) | % Change in Systolic Blood Pressure |
| dose 1 mg/kg | 1 | -27 ± 5 (6 rats) |
| Initial Blood | 2 | -24 ± 1 (6 rats) |
| Pressure 265 ± 8 mmHg | 4 | -19 ± 6 (6 rats) |
| | 6 | -25 ± 5 (6 rats) |

## Claims

1. A compound of formula (I):

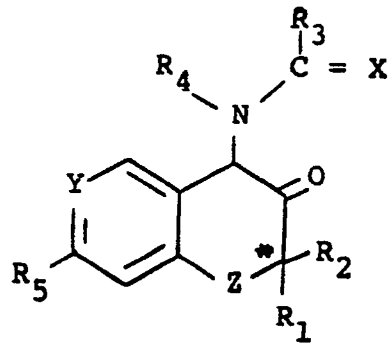

(I)

or, where appropriate, a pharmaceutically acceptable salt thereof, or a pharmaceutically acceptable solvate thereof,
characterised in that:
X represents oxygen or sulphur;
Y represents N or $N^{+}-O^{-}$ or a moiety $CR_6$ wherein $R_6$ is as defined below;
Z represents O, $-CH_2-$, $NR^o$, a bond or $S(O)_p$ wherein p represents zero, 1 or 2;
$R^o$ represents hydrogen, alkyl or alkylcarbonyl;
$R_1$ and $R_2$ independently represent hydrogen or alkyl; or
$R_1$ and $R_2$ together represent a $C_{2-7}$ polymethylene moiety;
$R_3$ represents hydrogen; alkyl optionally substituted by one or more groups or atoms selected from halogen, hydroxy or an ester thereof, alkoxy, alkoxycarbonyl, carboxy or an ester or amide thereof, amino, monoalkylamino or dialkylamino; alkenyl; amino optionally substituted by an alkyl or alkenyl group or by an alkanoyl group optionally substituted by up to three halo atoms, by a phenyl group optionally substituted by alkyl, alkoxy or halogen; substituted or unsubstituted aryl or substituted or unsubstituted heteroaryl; and
$R_4$ represents hydrogen or alkyl;
or $R_3$ and $R_4$ together represent a linking chain of formula $-A^1-A^2-$, $A^1$ being attached to the nitrogen atom of the moiety -N-CX- and $A^2$ being attached to the carbon atom of the group CX on the said moiety, and wherein $A^1$ represents a substituted or unsubstituted methylene group, $A^2$ represents 2 or 3 linking members, one of the linking members optionally representing O, S or NR and the other linking members each independently representing a substituted or unsubstituted methylene group;
R represents hydrogen, alkyl, alkanoyl, phenyl $C_{1-4}$-alkyl, arylcarbonyl wherein the aryl group may be substituted or unsubstituted; or R is mono- or bi-cyclic- heteroarylcarbonyl; or the moiety $R_4$.N.CX.$R_3$ forms a substituted or unsubstituted 2-pyridonyl group or a substituted or unsubstituted 2-thiopyridonyl group;
$R_5$ and $R_6$ are each independently selected from the class of hydrogen, substituted or unsubstituted alkyl, alkoxy, fluoroalkoxy, $C_{3-8}$ cycloalkyl, hydroxy or an ester thereof, nitro, cyano, halo, formyl, carboxy, a group of formula $R^aT^1$-, $R^bR^cNT$-, $R^aT^2NH$-, $R^dCO.O$-, $R^dCS.O$-, $R^d(OH)CH$-, $R^d(SH)CH$-, $R^dC(=N.OH)$-, $R^dC(=N.NH_2)$- or alkenyl optionally substituted by alkylcarbonyl, nitro or cyano, providing that when Y is $CR_6$ then at least one of $R_5$ or $R_6$ is not hydrogen;
$R^a$ represents $R^d$ or $R^dO$- and $R^d$ represents substituted or unsubstituted alkyl, substituted or unsubstituted aryl or substituted or unsubstituted heteroaryl, optional substituents for $R^d$ being up to 3 substituents selected from alkyl, alkoxy, halo, haloalkyl, nitro and cyano;
$R^b$ and $R^c$ each independently represent hydrogen, alkyl or alkylcarbonyl;
T represents a bond or $T^1$, $T^1$ represents -CS- or $T^2$ and
$T^2$ represents -CO-, -SO- or $-SO_2-$.

2. A compound according to claim 1, wherein Y is $CR_6$, one of $R_5$ and $R_6$ is hydrogen and the other is selected from the class of $C_{1-6}$ alkylcarbonyl, $C_{1-6}$ alkoxycarbonyl, $C_{1-6}$ alkylcarbonyloxy, $C_{1-6}$ alkylhydroxymethyl, nitro, cyano, chloro, trifluoromethyl, $C_{1-6}$ alkylsulphinyl, $C_{1-6}$ alkylsulphonyl, $C_{1-6}$ alkoxysulphinyl, $C_{1-6}$ alkoxysulphonyl, $C_{1-6}$ alkylcarbonylamino, $C_{1-6}$ alkoxycarbonylamino, $C_{1-6}$ alkyl-thiocarbonyl, $C_{1-6}$ alkoxy-thiocarbonyl, $C_{1-6}$ alkyl-thiocarbonyloxy, $C_{1-6}$ alkyl-thiomethyl, formyl or aminosulphinyl, aminosulphonyl or aminocarbonyl, the amino moiety being optionally substituted by one or two $C_{1-6}$ alkyl groups, or $C_{1-6}$ alkylsulphinylamino, $C_{1-6}$ alkylsulphonylamino $C_{1-6}$ alkoxysulphinylamino or $C_{1-6}$ alkoxysulphonylamino or ethenyl terminally substituted by $C_{1-6}$ alkylcarbonyl, nitro or cyano, or $-C(C_{1-6}$ alkyl)NOH or $-C(C_{1-6}$ alkyl)$NNH_2$.

3. A compound according to claim 1 or claim 2, wherein $R_5$ is hydrogen and $R_6$ is cyano.

4. A compound according to any one of claims 1 to 3, wherein Z represents O.

5. A compound according to any one of claims 1 to 4, wherein $R_1$ and $R_2$ are both methyl.

6. A compound according to any one of claims 1 to 5, wherein the moiety $R_3$.N.CX.$R_4$ represents either pyrrolidonyl or piperidonyl.

7. A compound according to claim 1, being 6-cyano-3,4-dihydro-2,2 dimethyl-4-(2-oxopyrrolidin-1-yl)-2H-1-benzopyran-3-one or a pharmaceutically acceptable salt thereof or a pharmaceutically acceptable solvate thereof.

8. A process for the preparation of a compound of formula (I) or, where appropriate a pharmaceutically acceptable salt thereof, or a pharmaceutically acceptable solvate thereof, which process comprises deprotecting a compound of formula (II):

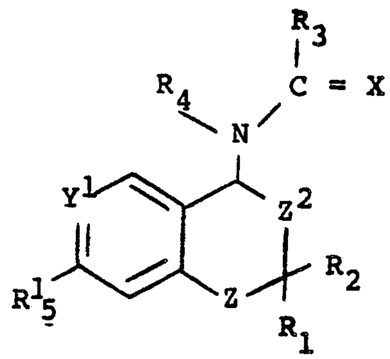

(II)

wherein $R_1$, $R_2$, $R_3$, $R_4$, X and Z are as defined in relation to formula (I), $R^1_5$ represents $R_5$ or a group or atom convertible thereto, $Y^1$ represents Y or a group convertible thereto and $Z^2$ represents a protected keto group; and thereafter, if required, carrying out one or more of the following optional steps:

(i) converting a compound of formula (I) into a further compound of formula (I):

(ii) converting $Y^1$ to Y and/or $R^1_5$ to $R_5$;

(iii) forming a pharmaceutically acceptable salt of the compound of formula (I) and/or a pharmaceutically acceptable solvate thereof.

9. A pharmaceutical composition which comprises a compound of formula (I), or a pharmaceutically acceptable salt thereof or a pharmaceutically acceptable solvate thereof, and a pharmaceutically acceptable carrier.

10. A compound of formula (I) or a pharmaceutically acceptable salt thereof, or a pharmaceutically acceptable solvate thereof, for use in the treatment of respiratory tract disorders or hypertension.

11. The use of a compound of formula (I), or a pharmaceutically acceptable salt thereof, or a pharmaceutically acceptable solvate thereof for the manufacture of a medicament for the treatment of respiratory tract disorders or hypertension.

European Patent Office

# EUROPEAN SEARCH REPORT

Application Number

EP 89 30 9927

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 214 818 (BEECHAM GROUP)<br>* page 1, claim 1; page 5, claims 9,10 * | 1,9 | C 07 D 311/68<br>C 07 D 217/04<br>C 07 D 217/08<br>C 07 D 405/04<br>C 07 D 407/04<br>C 07 D 491/052<br>C 07 D 491/04<br>A 61 K 31/44 //<br>(C 07 D 405/04<br>C 07 D 311:00<br>C 07 D 207:00 ) |
| A | EP-A-0 205 292 (BEECHAM GROUP)<br>* page 20, claim 1; page 21, claim 7; page 22, claims 9,10 * | 1,9 | |
| A,D | EP-A-0 176 689 (BEECHAM GROUP)<br>* the whole document * | 1,9 | |
| A | EP-A-0 168 619 (BEECHAM GROUP)<br>* page 1, claim 1; page 6, claims 9,10 * | 1,9 | |
| A | JOURNAL OF MEDICINAL CHEMISTRY vol. 29, no. 11, 1986, pages 2194-2201; J.M. EVANS et al.: "Synthesis and Antihypertensive Activity of 4-(Cyclic amido)-2H-1-benzopyrans" * the whole document * | 1,9 | **TECHNICAL FIELDS SEARCHED (Int. Cl.5)** |
| A | JOURNAL OF MEDICINE CHEMISTRY vol. 16, no. 4, 1973, pages 332-336; J.W. MATHISON et al.: "Synthesis and Hypotensive Properties of Tetrahydroisoquinolines" * the whole document * | 1,9 | C 07 D 311/00<br>C 07 D 217/00<br>C 07 D 405/00<br>C 07 D 407/00<br>C 07 D 491/00 |

The present search report has been drawn up for all claims

| Place of search | Date of completion of the search | Examiner |
|---|---|---|
| BERLIN | 11-12-1989 | KYRIAKAKOU G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)